# FASCICULE DE BREVET EUROPEEN

(11) **EP 0 754 448 B1**
(45) Date de publication et mention de la délivrance du brevet: **23.07.1997**
(21) Numéro de dépôt: 96401142.3
(22) Date de dépôt: 28.05.1996
(51) Int. Cl.: A61K 7/48

(54) **Composition sous forme de poudre auto-émulsionnable dans l'eau**
Mittel in Form eines in Wasser selbstemulgierenden Pulver
Composition in form of a, in water self-emulsifying, powder

(30) Priorité: 10.07.1995 FR 9508313
(43) Date de publication de la demande: 22.01.1997
(73) Titulaire: L'OREAL, 75008 Paris (FR)
(72) Inventeur: Khayat, Carine, 94210 La Varenne (FR); Bordeaux, Dominique, 91240 Saint Michel sur Orge (FR); Candau, Didier, 91950 Bievres (FR)
(74) Mandataire: Miszputen, Laurent

(56) Documents cités:
- AU-A- 557 932
- FR-A- 2 553 788
- STN, Serveur de Bases de Données, XP002015677
- STN, Serveur de Bases de Données, XP002015677; Karlsruhe, DE, Fichier Chemical Abstracts, vol. 104, nA 147412

## Description

La présente invention a trait à une composition sous forme de poudre autoémulsionnable dans l'eau à savoir susceptible de reconstituer, à température ambiante, une émulsion huile-dans-eau stable après réhydratation dans l'eau, à son procédé de préparation ainsi qu'à ses utilisations en cosmétique ou en dermatologie.

De nombreuses formulations cosmétiques pour le soin de la peau, actuellement sur le marché, sont des émulsions huile-dans-eau. Ces formes galléniques sont particulièrement recherchées pour leur confort à l'application, leur texture onctueuse, agréable, leur sensation de fraîcheur et de douceur sur la peau et du fait qu'elles peuvent être appliquées sur tous les types de peau.

Elles sont généralement constituées d'une phase aqueuse, d'une phase grasse et d'un tensio-actif émulsionnant pour stabiliser l'interface huile/eau.

Les tensio-actifs les plus couramment utilisés comme émulsionnants sont des molécules amphiphiles de petite taille constituées d'une partie lipophile généralement une chaîne grasse en C₈-C₂₂ et d'une partie hydrophile telle que des groupes oxydes d'éthylène, oxydes de propylène, des sorbitanes, des sucres, des groupes glycérolés. Ces tensio-actifs présentent un potentiel irritant vis-à-vis de la peau et notamment pour les peaux sensibles.

Au cours de ces dernières années, les macromolécules protéiques telles que les protéines de graine végétale : de céréale ou d'oléagineux et les protéines lactiques sont très recherchées en cosmétique pour leurs propriétés hydratantes, leur bonne tolérance vis-à-vis de la peau et du corps humain et du fait qu'elles conduisent à de nouvelles textures intéressantes au niveau cosmétique à savoir crémeuses et onctueuses, d'aspect brillant en conditionnement et d'aspect mat à l'application sur la peau ; elles confèrent un toucher particulièrement doux et non-gras.

La présente invention a donc pour objectif de réaliser des émulsions huile-dans-eau stables et homogènes contenant des macromolécules protéiques sans utiliser d'agent tensio-actif émulsionnant pour stabiliser l'interface huile/eau.

Or les macromolécules protéiques prises en tant que telles, en raison de leur structure globulaire, ne permettent pas de stabiliser l'interface huile/eau d'une émulsion. La partie lipophile constituée par des acides aminés, recroquevillée à l'intérieur de la structure globulaire de la protéïne est difficile à déployer à l'interface huile/eau.

Une des solutions pour résoudre ce problème serait d'augmenter le taux de protéines dans l'émulsion de manière à la stabiliser par encombrement stérique. Cette stabilisation conduirait à des compositions présentant un toucher collant indésirable sur le plan cosmétique et difficilement applicables sur la peau.

Une autre solution serait de dénaturer les protéines à l'interface huile/eau par un traitement de chauffage, par modification de pH ou par changement des forces ioniques notamment en ajoutant un sel.

Un des objectifs de l'invention est de pouvoir réaliser des compositions à base de protéines pouvant être stockées à sec sous la forme d'une poudre dispersible dans l'eau et permettant de reconstituer instantanément, par simple addition d'eau, à température ambiante, sans traitement de dénaturation des protéines, une émulsion huile-dans-eau à base de protéines, stable et prête à l'emploi.

D'autre part, les émulsions huile-dans-eau contenant des protéines nécessitent en général l'utilisation d'agents conservateurs pour éviter le développement des bactéries favorisé par la présence d'eau et de protéines. Il est souhaitable d'éliminer dans un produit cosmétique, la présence d'agents conservateurs pouvant être irritants pour des peaux sensibles et de pouvoir disposer, au moment de l'utilisation, d'une composition stable et saine.

La demanderesse a découvert de manière surprenante une composition sous forme de poudre à base de protéines et de lipides issus d'une même graine végétale et d'un polysaccharide, susceptible de reconstituer, après réhydratation dans l'eau, une émulsion huile-dans-eau stable sans qu'il soit nécessaire d'utiliser un tensio-actif émulsionnant ou de dénaturer les protéines par un traitement tel que mentionné ci-dessus.

Les compositions selon l'invention, sous forme de poudre sont autoémulsionnables et facilement dispersibles dans l'eau, à savoir qu'elles produisent instantanément, à température ambiante, par simple addition d'eau une émulsion huile-dans-eau. Elles sont stables au stockage pendant plusieurs mois dans les conditions normales de conservation et peuvent être utilisées au moment de l'utilisation sans conservateur.

Les compositions selon l'invention permettent d'obtenir après réhydration dans l'eau, des compositions cosmétiques ou dermatologiques, prêtes à l'emploi, sous forme d'émulsion huile-dans-eau ayant une bonne tolérance vis-à-vis de la peau et notamment des peaux sensibles. Les compositions cosmétiques ou dermatologiques ainsi reconstituées présentent une texture onctueuse originale, facilement applicable sur la peau, conférant un toucher particulièrement doux et non-gras sur la peau ainsi que des propriétés hydratantes.

Les compositions sous forme de poudre selon l'invention, du fait de leur bonne tolérance vis-à-vis du corps humain, peuvent être utilisées en cosmétique orale et être encapsulées dans des capsules ingérables.

Les compositions selon l'invention sont sous forme de poudre auto-émulsionnable dans l'eau et sont caractérisées par le fait qu'elles sont susceptibles d'être obtenues à partir d'une fraction liquide aqueuse, extraite d'une graine végétale, contenant des lipides et des protéines, que l'on sèche sur un support polysaccharidique.

Les graines végétales utilisées selon l'invention sont de préférence choisies parmi les amandes de noyau de fruit telles que des amandes de noyau de pêche ou d'abricot.

Elles sont plus préférentiellement dépelliculées afin d'obtenir des compositions incolores.

Les amandes de noyau d'abricot sont plus particulièrement utilisées selon l'invention.

Les supports de séchage polysaccharidiques utilisés selon l'invention sont choisis de préférence dans le groupe formé par :
- les farines de graines telles que la gomme de guar, la gomme de caroube ou l'amidon de maïs naturel ou modifié ;
- les exsudats d'arbres tels que la gomme arabique, la gomme karaya, la gomme adragante ou la gomme de ghatty ;
- les extraits d'algues tels que les carraghénates, les alginates et l'agar-agar ;
- les exsudats de micro-organismes tels que la gomme de xanthane, la cellulose et ses dérivés.
- les dextrines et les malto-dextrines.

Parmi ces polysaccharides, la gomme de xanthane est particulièrement préférée.

Les compositions selon l'invention sous forme de poudre présentent une humidité résiduelle allant, préférentiellement, de 1 à 8%.

Elles contiennent de préférence de 10 à 60% en poids de protéines issues de la graine végétale telle que définie ci-dessus et de 5 à 60% en poids de lipides issus de ladite graine végétale ; les pourcentages en poids étant calculés par rapport au poids total de la poudre.

Le support de séchage du type polysaccharide est présent dans les compositions de l'invention dans des concentrations allant, préférentiellement, de 0,1 à 50% en poids par rapport au poids total de la composition.

Les fractions liquides aqueuses extraites de graines végétales à partir desquelles sont obtenues les compositions de l'invention sont préparées selon un procédé comportant les étapes suivantes :
a) on broie les graines végétales dans de l'eau pour obtenir une dispersion aqueuse;
b) on effectue une extraction aqueuse de ladite dispersion ;
c) on sépare les corps insolubles de l'extrait aqueux ;
d) on isole la fraction contenant les protéines et les lipides de l'extrait aqueux ;
e) on homogénéise ladite fraction et on la stérilise.

Les graines végétales utilisées sont de préférence des amandes dépelliculées de noyau d'abricot.

On peut effectuer l'étape de broyage des amandes dans de l'eau à 10-35° C dans un rapport pondéral de 1/12 à 1/3. On peut utiliser un agitateur à haute vitesse de type POLYTRON® pour obtenir le broyage le plus fin possible.

Les amandes d'abricot peuvent être douces ou amères.

Dans le cas d'amandes douces, l'extraction aqueuse peut être effectuée à pH 5-11 à 10-50°C pendant 1 à 5 heures. On peut séparer les insolubles dudit extrait aqueux sur décanteur solide/liquide à l'aide d'une centrifugeuse, d'une essoreuse ou d'un filtre. On peut isoler la fraction contenant des protéines et des lipides, que l'on cherche à obtenir pour l'invention, par précipitation à pH 3-5 à 10-50°C et l'on peut écarter le surnageant riche en sucres par filtration tangentielle, ultrafiltration ou microfiltration.

Dans le cas d'amandes amères, l'extraction aqueuse peut être effectuée à pH 5-11 à 10-45°C pendant 1 à 10 heures. On peut séparer les insolubles, plus particulièrement l'acide cyanhydrique dudit extrait aqueux à l'aide d'une centrifugeuse, d'une essoreuse ou de filtres. On peut isoler la fraction liquide par ultrafiltration et/ou microfiltration et écarter ainsi le filtrat riche en sucres et en restes de cyamines.

L'extrait aqueux ou la fraction liquide à base de protéines et de lipides peuvent éventuellement subir un écrèmage à l'aide d'une centrifugeuse ou d'une écrémeuse à des températures allant de 4 à 60°C et de préférence de 35 à 45°C.

L'étape d'homogénéisation de la fraction liquide à base de protéines et de lipides peut être effectuée en un ou plusieurs passage(s) à 50-1000 bars à 45-85°C, de préférence à 200-350 bars à 45-65°C.

On peut stériliser ladite fraction à 80-160°C pendant 5 secondes à 60 minutes, de préférencede 130-150°C pendant 20-80 s.

La fraction liquide, utilisée pour l'obtention des compositions selon l'invention, contient de préférence :
a) de 10 à 60 % en poids de protéines issues de la graine végétale ;
b) de 5 à 60 % en poids de lipides issus de la même graine végétale ;
c) et d'eau.

Un autre objet de l'invention consiste en un procédé de préparation des compositions sous forme de poudre auto-émulsionnable dans l'eau telles que décrites ci-dessus.

Le procédé de l'invention est caractérisée par le fait que l'on mélange la fraction liquide, extraite de graine végétale, à base de protéines et de lipides telle que définie précédemment avec un polysaccharide tel que défini précédemment, que l'on homogénéise le mélange obtenu et que l'on sèche pour obtenir une poudre.

L'étape d'homogénéisation se fait dans les mêmes conditions que celle de la fraction liquide définie précédemment.

L'étape de séchage est effectuée selon une méthode classique telle que par pulvérisation dans un courant d'air chaud comme l'atomisation ou le séchage par sublimation comme la lyophilisation. La teneur en eau résiduelle doit être de préférence comprise entre 1 et 8%.

On obtient ainsi une poudre facilement dispersible dans l'eau et capable de reconstituer, à température ambiante, par addition de liquide aqueux, instantanément une émulsion huile-dans-eau stable, homogène sans séparation de phase.

Un autre objet de l'invention est l'utilisation de la composition sous forme de poudre pour la reconstitution d'émulsions huile-dans-eau cosmétiques ou dermatologiques. L'utilisation de la poudre dépendra de la quantité de liquide ajouté pour reconstituer l'émulsion et de l'application envisagée. On obtiendra, selon la quantité d'eau ajoutée, des compositions plus ou moins épaissies. Les compositions cosmétiques ainsi obtenues auront une consistance plus ferme pour une crème de soin ou une crème hydratante et plus ou moins liquide pour un lait démaquillant, une huile de soin ou un shampooing.

De façon préférentielle, par addition de liquide aqueux dans un rapport poudre/liquide allant de 10/90 à 60/40, on peut reconstituer un produit cosmétique ou dermatologique classique sous forme d'émulsion huile-dans-eau.

Il est possible d'ajouter à la poudre, après reconstitution, d'autres ingrédients cosmétiques ou dermatologiques classiques choisis selon l'application envisagée.

Un autre objet de l'invention est une utilisation des compositions sous forme de poudre telles que définies précédemment dans des capsules ingérables cosmétiques utilisables comme compléments diététiques.

Les exemples qui suivent servent à illustrer l'invention.

### EXEMPLES

### EXEMPLE DE PREPARATION 1:

On concasse à sec 50 kg d'amandes douces d'abricots dépelliculées présentant une teneur en matière sèche de 96%, une teneur en proteïnes de 27,5% (13,2kg) et une teneur en lipides de 52% (25 kg).

On broie ces amandes dans 400 kg d'eau à 25°C dans un agitateur à haute vitesse POLYTRON®. On pompe directement la dispersion aqueuse ainsi obtenue dans un moulin colloïdal où on la soumet à un deuxième broyage de manière à obtenir une dispersion aqueuse particulièrement fine.

On effectue sur cette dispersion une extraction aqueuse à pH 7 à 25°C pendant 2 heures.

On écarte les insolubles de l'extrait aqueux présentant par centrifugation à 25°C pendant 3 heures.

On recueille 360 kg d'extrait aqueux présentant une teneur en matière sèche de 8,9% et contenant 72,4% des protéines et 74,2% des lipides des amandes douces d'abricots dépelliculées de départ.

A partir de 65 kg de cet extrait aqueux, on isole une fraction riche en protéines et en lipides par précipitation à pH 4 à 25°C et l'on écarte le surnageant riche en sucres par centrifugation à 25°C.

On recueille ainsi 19,6 kg d'une fraction présentant une matière sèche de 11,9% et contenant 57,1% des protéines et 38% des lipides contenus dans les 65 kg d'extrait aqueux.

On ajuste la teneur en matière sèche de cette fraction à 10% par addition d'eau et l'on ajuste son pH à 7.

On ajoute de la gomme de xanthane comme support de séchage à 11,3 kg de la fraction diluée, à raison de 5% en poids sur la matière sèche de la fraction.

On homogénéise la fraction diluée à 50°C dans un homogénéisateur RANNIE® en deux passages successifs, le premier à 250 bars et le second à 50 bars. La fraction homogéneisée ainsi obtenue présente une texture onctueuse.

On stérilise la fraction homogénéisée par chauffage à 140°C pendant 40 s.

On sèche la fraction stérilisée dans un atomiseur NIRO^{®}, avec une vitesse d'atomisation de 22000 tours/minute, une température d'entrée d'air de 170°C et une température de sortie du produit de 80°C.

On obtient une poudre autoémulsionnable présentant les caractéristiques suivantes :

| | |
|---|---|
| Teneur en protéines | 39,3% en poids |
| Teneur en lipides | 51,3% en poids |
| Teneur en gomme de xanthane | 5,0% en poids |
| Teneur en eau | 2,2% en poids |

### EXEMPLE DE PREPARATION 2:

On procède de la manière décrite à l'exemple de préparation 1 jusqu'à l'obtention de l'extrait aqueux dont on a écarté les insolubles.

On recueille 260 kg d'extrait aqueux riche en protéines et en lipides présentant une teneur en matière sèche de 8,9% et contenant 72,4% des protéines et 74,2% des lipides des amandes douces d'abricots dépelliculées de départ.

On écrème ces 260 kg d'extrait aqueux riche en protéines et en lipides à l'aide d'une centrifugeuse à 40°C.

D'une part, on recueille 21,8 kg de crème contenant 6,2% des protéines et 95% des lipides contenus dans les 260 kg de l'extrait aqueux soit 1,5% des protéines et 48,2% des lipides des amandes douces dépelliculées de départ. On ajuste la teneur en matière sèche de cette crème à 50,5% par addition d'eau et on la conserve à 4°C.

D'autre part, on recueille 227 kg d'extrait aqueux riche en protéines et en lipides présentant une matière sèche de 3,7% et contenant 81,9% des protéines et 1,4% des lipides contenus dans les 260 kg d'extrait aqueux riche en protéines et en lipides.

A partir de 160 kg de cet extrait aqueux, on isole une fraction riche en protéines et en lipides par précipitation à pH 4 à 25°C et l'on écarte le surnageant riche en sucres par centrifugation à 25°C.

La fraction obtenue présente une teneur en matière sèche de 12,5% et contient 86,4% des protéines contenues dans les 160 kg d'extrait aqueux enrichi. On ajuste la teneur en matière sèche de cette fraction à 10% par addition d'eau et l'on ajuste son pH à 7.

On reconstitue une nouvelle fraction enrichie en lipides et protéines à partir de la crème obtenue précédemment et de la fraction obtenue en dernier lieu.

On mélange 1,1 kg de crème avec 12 kg de fraction enrichie. On ajoute de la gomme de xanthane comme support de sèchage à 13,1 kg de la fraction ainsi reconstituée, à raison de 5% en poids sur la matière sèche de ladite fraction reconstituée.

On homogénéise la fraction reconstituée à 50°C dans un homogénéisateur RANNIE® en deux passages successifs, le premier à 250 bars et le second à 50 bars.

On stérilise la fraction reconstituée homogénéisée par chauffage à 140°C pendant 40 s.

On sèche la fraction reconstituée stérilisée dans un atomiseur NIRO^{®}, avec une vitesse d'atomisation de 22000 tours/minute, une température d'entrée d'air de 170°C et une température de sortie du produit de 80°C.

On obtient une poudre autoémulsionnable présentant les caractéristiques suivantes :

| | |
|---|---|
| Teneur en protéines | 57,7% en poids |
| Teneur en lipides | 31,2% en poids |
| Teneur en gomme de xanthane | 5,0% en poids |
| Teneur en eau | 4,5% en poids |

### EXEMPLE DE PREPARATION 3 :

On concasse à sec 50 kg d'amandes amères d'abricots dépelliculées présentant une teneur en matière sèche de 96%, une teneur en proteïnes de 26% (12,5 kg) et une teneur en lipides de 47% (22,6 kg).

On broie ces amandes dans 400 kg d'eau à 25°C dans un agitateur à haute vitesse POLYTRON®. On pompe directement la dispersion aqueuse ainsi obtenue dans un moulin colloïdal où on la soumet à un deuxième broyage de manière à obtenir une dispersion aqueuse particulièrement fine.

On effectue sur cette dispersion une extraction aqueuse à pH 7 à 25°C pendant 2 heures.

On écarte les insolubles de l'extrait aqueux présentant par centrifugation à 25°C pendant 3 heures.

On recueille 331,9 kg d'extrait aqueux présentant une teneur en matière sèche de 10,3% et contenant 60,4% des protéines et 66,3% des lipides des amandes amères d'abricots dépelliculées de départ.

A partir de cet extrait aqueux, on isole une fraction riche en protéines et en lipides par ultrafiltration à 50°C et l'on écarte le filtrat riche en sucres et en restes de cyanure.

On recueille ainsi 65,7 kg d'une fraction présentant une teneur en matière sèche de 22,7% et contenant 11,8% des protéines et 92,7% des lipides contenus dans les 331,9 kg d'extrait aqueux.

On écrème 50 kg de cette fraction riche en protéines et en lipides à l'aide d'une centrifugeuse à 40°C.

D'une part, on recueille 16,7 kg de crème présentant une teneur en matière sèche de 61,4% et contenant 36,7% des protéines et 89,5% des lipides contenus dans la fraction riche en protéines et lipides.

D'autre part, on recueille 29,7 kg de fraction riche en protéines présentant une matière sèche de 2,66% et contenant 41,9% des protéines et 1,7% des lipides contenus dans la fraction riche en protéines et lipides.

On ajoute de l'amidon de maïs comme support de séchage aux 29,7 kg de la fraction riche en protéines, à raison de 50% en poids sur la matière sèche de ladite fraction.

On homogénéise la fraction obtenue, à 50°C dans un homogénéisateur RANNIE® en deux passages successifs, le premier à 250 bars et le second à 50 bars.

On stérilise la fraction homogénéisée par chauffage à 140°C pendant 40 s.

On sèche la fraction stérilisée dans un atomiseur NIRO^{®}, avec une vitesse d'atomisation de 22000 tours/minute, une température d'entrée d'air de 170°C et une température de sortie du produit de 80°C.

On obtient une poudre autoémulsionnable présentant les caractéristiques suivantes :

| | |
|---|---|
| Teneur en protéines | 21,4% en poids |
| Teneur en lipides | 18,4% en poids |
| Teneur en amidon de maïs | 50,0% en poids |
| Teneur en eau | 7,6% en poids |

### EXEMPLE A : Crème de jour hydratante

| | |
|---|---|
| Poudre autoémulsionnable de l'exemple 1 | 20% en poids |
| Eau | 80% en poids |

### Mode opératoire :

Le mélange se fait à température ambiante par simple agitation dans un récipient fermé et reste stable au moins 15 jours. On obtient une crème d'aspect riche et brillant donnant à l'application un final mat et doux.

### EXEMPLE B : Lait corporel hydratant

| | |
|---|---|
| Poudre autoémulsionnable de l'exemple 2 | 10% en poids |
| Eau | 90% en poids |

### Mode opératoire :

Le mélange se fait à température ambiante par simple agitation dans un récipient fermé et reste stable au moins 7 jours. On obtient une crème d'aspect riche et brillant donnant à l'application un final mat et doux.

### EXEMPLE C : Masque hydratant

| | |
|---|---|
| Poudre autoémulsionnable de l'exemple 3 | 40% en poids |
| Eau | 60% en poids |

### Mode opératoire :

Le mélange se fait à température ambiante par simple agitation dans un récipient fermé et reste stable au moins 15 jours. On obtient une crème d'aspect riche et brillant donnant à l'application un final mat et doux.

### EXEMPLE D: Capsules ingérables cosmétiques

| | |
|---|---|
| Poudre autoémulsionnable de l'exemple 3 | 95% en poids |
| Vitamine C en poudre | 5% en poids |

### Mode opératoire :

Le mélange des deux poudres se fait dans un mélangeur classique type BAKER®. La poudre est ensuite incorporée dans des petites capsules ingérables. On obtient un support anhydre de la vitamine C dans lequel celle-ci est stable. Les capsules redonnent de l'éclat au teint.

## Revendications

1. Composition sous forme de poudre autoémulsionnable dans l'eau après réhydratation, caractérisée par le fait qu'elle est susceptible d'être obtenue à partir d'une fraction liquide aqueuse, extraite d'une graine végétale, contenant des protéines et des lipides, que l'on déshydrate par séchage sur un support polysaccharidique.

2. Composition selon la revendication 1, caractérisée par le fait que la graine végétale est une amande de noyau de fruit.

3. Composition selon la revendication 2, caractérisée par le fait que la graine végétale est une amande de noyau de fruit dépelliculée.

4. Composition selon l'une quelconque des revendications 1 à 3, caractérisée par le fait que la fraction liquide à base de protéines et de lipides est extraite d'amande de noyau d'abricot.

5. Composition selon l'une quelconque des revendications 1 à 4, caractérisée par le fait que le support de séchage polysaccharidique est choisi dans le groupe formé par les extraits d'algues, les farines de graines, les exsudats d'arbre et les exsudats de micro-organismes, les dextrines et les malto-dextrines.

6. Composition selon la revendication 5, caractérisée par le fait que le support de séchage est la gomme de xanthane.

7. Composition selon l'une quelconque des revendications 1 à 6, caractérisée par le fait qu'elle a une teneur en eau résiduelle allant de 1 à 8 %.

8. Composition selon l'une quelconque des revendications 1 à 7, caractérisée par le fait qu'elle contient :
a) de 10 à 60 % en poids de protéines issues de la graine végétale ;
b) de 5 à 60 % en poids de lipides issus de la graine végétale ;
c) de 0,1 à 50 % en poids de support polysaccharidique ;
les pourcentages en poids sont calculés par rapport au poids total de la composition.

9. Procédé de préparation d'une poudre autoémulsionnable dans l'eau après réhydratation, caractérisé par le fait que l'on mélange une fraction liquide aqueuse contenant des protéines et des lipides, extraite d'une graine végétale avec un polysaccharide, que l'on homogénéise le mélange obtenu et que l'on sèche pour obtenir une poudre.

10. Procédé selon la revendication 9, caractérisé par le fait que le séchage est effectuée par atomisation ou lyophilisation.

11. Procédé selon la revendication 9 ou 10, caractérisé par le fait que la graine végétale est une amande de noyau de fruit.

12. Procédé selon l'une quelconque des revendications 9 à 11, caractérisé par le fait que la graine végétale est une amande de noyau de fruit dépelliculée.

13. Procédé selon l'une quelconque des revendications 9 à 12, caractérisé par le fait que la graine végétale est une amande de noyau d'abricot.

14. Procédé selon l'une quelconque des revendications 9 à 13, caractérisé par le fait que le polysaccharide est choisi dans le groupe formé par les extraits d'algues, les farines de graines, les exsudats d'arbre, les exsudats de micro-organismes, les dextrines et les malto-dextrines.

15. Procédé selon la revendication 14, caractérisé par le fait que le polysaccharide est la gomme de xanthane.

16. Procédé selon l'une quelconque des revendications 9 à 15, caractérisé par le fait que le séchage est effectué jusqu'à une teneur en eau résiduelle comprise entre 1 et 8 %.

17. Procédé selon l'une quelconque des revendications 9.à 16, caractérisé par le fait que la fraction liquide aqueuse extraite de graine végétale contient :
a) de 10 à 60 % en poids de protéines issues de la graine végétale,
b) de 5 à 60 % en poids de lipides issus de la graine végétale ; les pourcentages en poids sont données par rapport au poids de la fraction.

18. Procédé selon l'une quelconque des revendications 9 à 17, caractérisé par le fait que le polysaccharide est utilisé dans des quantités allant de 0,1 à 50 % en poids par rapport au poids total de la poudre.

19. Utilisation de la composition selon l'une quelconque des revendications 1 à 8, pour la préparation d'une composition cosmétique ou dermatologique sous forme d'émulsion huile-dans-eau que l'on reconstitue par addition d'un liquide aqueux.

20. Utilisation selon la revendication 19, caractérisée par le fait que le rapport en poids poudre/liquide varie de 10/90 à 60/40.

21. Utilisation de la composition selon l'une quelconque des revendications 1 à 8, pour la fabrication d'un produit cosmétique ou dermatologique, sous forme de capsules ingérables, selon laquelle ladite composition est encapsulée dans lesdites capsules.

## Claims

1. Composition in the form of a powder which is self-emulsifiable in water after rehydration, characterized in that it may be obtained from an aqueous liquid fraction extracted from a plant seed, containing proteins and lipids, which is dehydrated by drying on a polysaccharide support.

2. Composition according to Claim 1, characterized in that the plant seed is a kernel from a fruit stone.

3. Composition according to Claim 2, characterized in that the plant seed is a decorticated kernel from a fruit stone.

4. Composition according to any one of Claims 1 to 3, characterized in that the liquid fraction based on proteins and lipids is extracted from apricot stone kernel.

5. Composition according to any one of Claims 1 to 4, characterized in that the polysaccharide drying support is chosen from the group formed by algal extracts, seed flours, tree exudates and microorganism exudates, dextrins and maltodextrins.

6. Composition according to Claim 5, characterized in that the drying support is xanthan gum.

7. Composition according to any one of Claims 1 to 6, characterized in that it has a residual water content ranging from 1 to 8%.

8. Composition according to any one of Claims 1 to 7, characterized in that it contains:
a) from 10 to 60% by weight of proteins obtained from the plant seed;
b) from 5 to 60% by weight of lipids obtained from the plant seed;
c) from 0.1 to 50% by weight of polysaccharide support;
the percentages by weight are calculated relative to the total weight of the composition.

9. Process for the preparation of a powder which is self-emulsifiable in water after rehydration, characterized in that an aqueous liquid fraction containing proteins and lipids, extracted from a plant seed, is mixed with a polysaccharide and the mixture obtained is homogenized and dried in order to obtain a powder.

10. Process according to Claim 9, characterized in that the drying is carried out by spraying or freeze-drying.

11. Process according to Claim 9 or 10, characterized in that the plant seed is a kernel from a fruit stone.

12. Process according to any one of Claims 9 to 11, characterized in that the plant seed is a decorticated kernel from a fruit stone.

13. Process according to any one of Claims 9 to 12, characterized in that the plant seed is a kernel from an apricot stone.

14. Process according to any one of Claims 9 to 13, characterized in that the polysaccharide is chosen from the group formed by algal extracts, seed flours, tree exudates, microorganism exudates, dextrins and maltodextrins.

15. Process according to Claim 14, characterized in that the polysaccharide is xanthan gum.

16. Process according to any one of Claims 9 to 15, characterized in that the drying is carried out down to a residual water content of between 1 and 8%.

17. Process according to any one of Claims 9 to 16, characterized in that the aqueous liquid fraction extracted from plant seed contains:
a) from 10 to 60% by weight of proteins obtained from the plant seed,
b) from 5 to 60% by weight of lipids obtained from the plant seed; the percentages by weight are given relative to the weight of the fraction.

18. Process according to any one of Claims 9 to 17, characterized in that the polysaccharide is used in amounts ranging from 0.1 to 50% by weight relative to the total weight of the powder.

19. Use of the composition according to any one of Claims 1 to 8, for the preparation of a cosmetic or dermatological composition in the form of an oil-in-water emulsion which is reconstituted by addition of an aqueous liquid.

20. Use according to Claim 19, characterized in that the powder/liquid weight ratio ranges from 10/90 to 60/40.

21. Use of the composition according to any one of Claims 1 to 8, for the manufacture of a cosmetic or dermatological product, in the form of ingestible capsules, according to which the said composition is encapsulated in the said capsules.

## Patentansprüche

1. Zusammensetzung in Form von Pulver, die in Wasser nach Hydratisierung selbstemulgierfähig ist,
dadurch gekennzeichnet, daß
sie aus einer wäßrigen flüssigen Fraktion hergestellt werden kann, die aus einem Pflanzensamenkorn extrahiert wurde und Proteine und Lipide enthält, wobei durch Trocknen auf einem Polysaccharidträger das Wasser entzogen wird.

2. Zusammensetzung nach Anspruch 1, dadurch gekennzeichnet, daß das Pflanzensamenkorn ein Fruchtsamenkern ist.

3. Zusammensetzung nach Anspruch 2, dadurch gekennzeichnet, daß das Pflanzensamenkorn ein enthäuteter Fruchtsamenkern ist.

4. Zusammensetzung nach einem der Ansprüche 1 bis 3, dadurch gekennzeichnet, daß die flüssige Fraktion auf der Basis von Proteinen und Lipiden aus Aprikosensamenkernen gewonnen ist.

5. Zusammensetzung nach einem der Ansprüche 1 bis 4, dadurch gekennzeichnet, daß der Polysaccharidträger zum Trocknen unter den Algenextrakten, Mehlen von Samenkörnern, Baumexsudaten und Exsudaten von Mikroorganismen, Dextrinen und Maltodextrinen ausgewählt ist.

6. Zusammensetzung nach Anspruch 5, dadurch gekennzeichnet, daß der Träger zum Trocknen Xanthangummi ist.

7. Zusammensetzung nach einem der Ansprüche 1 bis 6, dadurch gekennzeichnet, daß sie einen Restwassergehalt von 1 bis 8 % aufweist.

8. Zusammensetzung nach einem der Ansprüche 1 bis 7, dadurch gekennzeichnet, daß sie enthält:
a) 10 bis 60 Gew.-% Proteine, die aus dem Pflanzensamenkorn stammen;
b) 5 bis 60 Gew.-% Lipide, die aus dem Pflanzensamenkorn stammen;
c) 0,1 bis 50 Gew.-% Polysaccharidträger;
die gewichtsbezogenen Prozentzahlen werden bezogen auf das Gesamtgewicht der Zusammensetzung berechnet.

9. Verfahren zur Herstellung eines Pulvers, das in Wasser nach Hydratisierung selbstemulgierfähig ist, dadurch gekennzeichnet, daß eine wäßrige flüssige Fraktion, die Proteine und Lipide enthält und aus einem Pflanzensamenkorn gewonnen ist, mit einem Polysaccharid vermischt wird, das hergestellte Gemisch homogenisiert und zur Herstellung eines Pulvers getrocknet wird.

10. Verfahren nach Anspruch 9, dadurch gekennzeichnet, daß die Trocknung durch Atomisierung oder Lyophilisation durchgeführt wird.

11. Verfahren nach Anspruch 9 oder 10, dadurch gekennzeichnet, daß das Pflanzensamenkorn ein Fruchtsamenkern ist.

12. Verfahren nach einem der Ansprüche 9 bis 11, dadurch gekennzeichnet, daß das Pflanzenkorn ein enthäuteter Fruchtsamenkern ist.

13. Verfahren nach einem der Ansprüche 9 bis 12, dadurch gekennzeichnet, daß das Pflanzensamenkorn ein Aprikosensamenkern ist.

14. Verfahren nach einem der Ansprüche 9 bis 13, dadurch gekennzeichnet, daß das Polysaccharid unter Algenextrakten, Mehlen von Samenkörnern, Baumexsudaten, Exsudaten von Mikroorganismen, Dextrinen und Maltodextrinen ausgewählt ist.

15. Verfahren nach Anspruch 14, dadurch gekennzeichnet, daß das Polysaccharid Xanthangummi ist.

16. Verfahren nach einem der Ansprüche 9 bis 15, dadurch gekennzeichnet, daß das Trocknen bis zu einem Restwassergehalt im Bereich von 1 bis 8 % durchgeführt wird.

17. Verfahren nach einem der Ansprüche 9 bis 16, dadurch gekennzeichnet, daß die flüssige wäßrige Fraktion, die aus Pflanzensamenkörnern extrahiert wurde, enthält:
a) 10 bis 60 Gew.-% Proteine, die aus dem Pflanzensamenkorn stammen,
b) 5 bis 60 Gew.-% Lipide, die aus dem Pflanzensamenkorn stammen,
wobei die gewichtsbezogenen Prozentzahlen bezogen auf das Gewicht der Fraktion angegeben sind.

18. Verfahren nach einem der Ansprüche 9 bis 17, dadurch gekennzeichnet, daß das Polysaccharid in Mengenanteilen im Bereich von 0,1 bis 50 Gew.-%, bezogen auf das Gesamtgewicht des Pulvers, verwendet wird.

19. Verwendung der Zusammensetzung nach einem der Ansprüche 1 bis 8 zur Herstellung einer kosmetischen oder dermatologischen Zusammensetzung in Form einer Öl-in-Wasser-Emulsion, die durch Zusatz einer wäßrigen Flüssigkeit gebildet wird.

20. Verwendung nach Anspruch 19, dadurch gekennzeichnet, daß das gewichtsbezogene Verhältnis von Pulver zu Flüssigkeit im Bereich von 10:90 bis 60:40 liegt.

21. Verwendung der Zusammensetzung nach einem der Ansprüche 1 bis 8 zur Herstellung eines kosmetischen oder dermatologischen Produkts in Form von Kapseln, die eingenommen werden können, wobei die Zusammensetzung in den Kapseln eingekapselt ist.
